# EUROPÄISCHE PATENTSCHRIFT

(11) **EP 4 204 514 B1**
(45) Veröffentlichungstag und Bekanntmachung des Hinweises auf die Patenterteilung: **06.08.2025**
(21) Anmeldenummer: 21769675.6
(22) Anmeldetag: 23.08.2021
(51) Int. Cl.: C09K 9/02, A61M 5/48, A61M 5/31, A61M 5/315

(54) **MEDIZINISCHE VORRICHTUNG ZUM VISUELLEN DARSTELLEN EINES INJEKTIONSDRUCKES EINES FLUIDES**
MEDICAL DEVICE FOR VISUALLY DISPLAYING AN INJECTION PRESSURE OF A FLUID
DISPOSITIF MÉDICAL PERMETTANT LA REPRÉSENTATION VISUELLE D'UNE PRESSION D'INJECTION D'UN FLUIDE

(30) Priorität: 25.08.2020 DE 102020210756
(43) Veröffentlichungstag der Anmeldung: 05.07.2023
(73) Patentinhaber: B. Braun Melsungen AG, 34212 Melsungen (DE)
(72) Erfinder: BERGER-ROSCHER, Nikolaus, 34127 Kassel (DE)
(74) Vertreter: Patentanwälte Ruff, Wilhelm, Beier, Dauster & Partner mbB
(86) Internationale Anmeldenummer: PCT/EP2021/073301
(87) Internationale Veröffentlichungsnummer: WO 2022/043276

(56) Entgegenhaltungen:
- WO-A1-2018/170231
- US-A1- 2012 109 071
- US-A1- 2013 150 785
- US-A1- 2014 069 202
- US-A1- 2017 354 403

## Beschreibung

### ANWENDUNGSGEBIET UND STAND DER TECHNIK

Die Erfindung betrifft eine medizinische Vorrichtung zum visuellen oder optischen Darstellen eines Injektionsdruckes eines Fluides, insbesondere medizinischen Fluides.

Viele medizinische Eingriffe sind ohne eine entsprechende Anästhesie nicht durchführbar. Man unterscheidet zwischen der Allgemeinanästhesie und der Lokalanästhesie. Den bedeutsamsten Teil der Lokalanästhesie stellt die sogenannte Regionalanästhesie, insbesondere periphere Regionalanästhesie, dar.

In der Regionalanästhesie wird ein Anästhetikum nahe desjenigen Nervs, der für die Reizweiterleitung von sogenannten Nozizeptoren verantwortlich ist, die im Bereich eines geplanten medizinischen Eingriffs liegen, verabreicht. Hierzu kommt in der Regel eine spezielle Kanüle zum Einsatz. Wird die Kanüle zu nahe an die Nerven oder in die Nerven geführt, können temporäre oder sogar permanente Nervenschädigungen entstehen. Derartige Nervenschädigungen können auch bei einem zu hohen Injektionsdruck des Anästhetikums auftreten. Daher sind sowohl die Positionierung der Kanüle als auch der Injektionsdruck entscheidend bei der Durchführung einer (peripheren) Regionalanästhesie.

Eine Vorrichtung zum visuellen Darstellen des Druckes eines medizinischen Fluides, wie insbesondere eines Anästhetikums, ist beispielsweise aus der WO 2003/101526 A1 bekannt. Die Vorrichtung wird zwischen einer Spritze und einer Kanüle eingesetzt. Die Vorrichtung besitzt einen die Spritze mit der Kanüle verbindenden Durchflusskanal, der mit einer Druckkammer kommuniziert. Die Druckkammer ist durch eine Membran begrenzt. Steigt der Injektionsdruck in dem Durchflusskanal und damit in der Druckkammer an, so wird die Membran ausgelenkt und drückt einen Anzeigestift gegen die Kraft einer Feder, so dass dieser Anzeigestift entsprechend dem in dem Durchflusskanal herrschenden Injektionsdruck unterschiedlich weit aus dem Gehäuse der Vorrichtung herausgeschoben wird, wodurch über eine Farbringcodierung des Anzeigestifts der Injektionsdruck visuell überwacht werden kann.

Eine Vorrichtung zur Begrenzung des Injektionsdruckes eines medizinischen Instruments zum Einbringen eines Fluides ist aus der WO 2017/071833 A1 bekannt. Bei dieser Vorrichtung wird der Injektionsdruck über ein Ventil begrenzt. Bei Erreichen eines Schwelldrucks wird die weitere Fluidzufuhr unterbrochen. Die Gegenkraft, die zum Schließen des Ventils erforderlich ist, wird ebenfalls durch eine Feder bewirkt.

Ferner ist aus der WO 2018/170231 A1 eine Injektionsspritze mit einem piezochromen Druckindikator bekannt, der über eine Farbänderung den Druck eines aus der Injektionsspritze ausgetragenen Fluides anzeigt.

Vergleichsweise komplexe Vorrichtungen zum Messen eines Drucks von medizinischen Fluiden sind aus den Druckschriften US 2013/0165904 A1 sowie US 2018/0064870 A1 bekannt.

Aus der US 2014/0069202 A1 ist eine medizinische Vorrichtung in Form einer Spritze mit einem Spritzenzylinder bekannt, wobei der Spritzenzylinder ein piezochromes Material enthalten kann.

Konventionelle Vorrichtungen zum visuellen Darstellen eines Injektionsdruckes von Fluiden weisen häufig den Nachteil auf, dass sie entweder einen sehr komplexen Aufbau besitzen oder farbige Druckindikatoren aufweisen, die entweder erst bei höheren Drücken ansprechen und/oder ein deutlich verzögertes Druckansprechverhalten besitzen, wodurch ihr Einsatz für medizinische, insbesondere regionalanästhetische, Anwendungen stark limitiert ist.

### AUFGABE UND LÖSUNG

Der Erfindung liegt die Aufgabe zugrunde, eine Vorrichtung zum visuellen oder optischen Darstellen eines Injektionsdruckes eines Fluides, insbesondere medizinischen Fluides wie Anästhetikums, bereitzustellen, die die einleitend im Zusammenhang konventioneller Vorrichtungen beschriebenen Nachteile wenigstens teilweise oder sogar vollständig umgeht.

Diese Aufgabe wird gelöst durch eine medizinische Vorrichtung gemäß Anspruch 1. Bevorzugte Ausgestaltungen der Erfindung sind Gegenstand der abhängigen Ansprüche. Der Wortlaut sämtlicher Ansprüche wird hiermit durch ausdrückliche Bezugnahme zum Inhalt der Beschreibung gemacht.

Die Erfindung betrifft eine medizinische Vorrichtung zum visuellen oder optischen Darstellen eines Injektionsdruckes, insbesondere Injektionsschwelldruckes, eines Fluides, vorzugsweise medizinischen Fluides, insbesondere Anästhetikums oder einer anästhetikumhaltigen Flüssigkeit.

Die medizinische Vorrichtung weist einen Fluidpfad für das Fluid, d.h. einen Fluidpfad, durch den das Fluid befördert wird, sowie ein piezochromes Material auf.

Die Erfindung zeichnet sich besonders dadurch aus, dass das piezochrome Material bei Einwirkung eines Drucks von < (gesprochen: kleiner) 100 bar eine für das menschliche Auge wahrnehmbare piezochrome Farbänderung zeigt.

Unter dem Ausdruck "Injektionsdruck" soll im Sinne der vorliegenden Erfindung ein Druck, vorzugsweise ein statischer Druck, verstanden werden, den ein Fluid bei dessen Injektion in den Körper eines Patienten auf Körperzellen, insbesondere Nervenzellen, und/oder Körpergewebe, insbesondere Nervengewebe, und/oder andere Körperstrukturen ausübt.

Unter dem Ausdruck "Druck" soll im Sinne der vorliegenden Erfindung ein Absolutdruck verstanden werden, d. h. ein Druck, der auf einen Druck Null, der in einem luftleeren Raum bzw. in einem Vakuum, insbesondere des Universums, herrscht, bezogen ist.

Unter dem Ausdruck "Injektionsschwelldruck" soll im Sinne der vorliegenden Erfindung ein Druck-Schwellenwert, vorzugsweise ein Schwellenwert eines statischen Druckes, verstanden werden, dessen Überschreitung unter vorzugsweise medizinischen Gesichtspunkten, insbesondere unter dem Gesichtspunkt einer Zellenschädigung, insbesondere Nervenzellenschädigung, und/oder einer Körpergewebeschädigung, insbesondere Nervengewebeschädigung, möglichst vermieden werden soll.

Unter dem Ausdruck "Fluid" soll im Sinne der vorliegenden Erfindung eine Flüssigkeit, vorzugsweise medizinische Flüssigkeit, insbesondere ein Anästhetikum oder eine anästhetikumhaltige Flüssigkeit, verstanden werden.

Unter dem Ausdruck "piezochromes Material" soll im Sinne der vorliegenden Erfindung ein Material verstanden werden, das seine Farbe bei Einwirkung von Druck ändert.

Die Erfindung basiert auf der überraschenden Erkenntnis, dass ein piezochromes Material, das seine Farbe bei Einwirkung eines Drucks von < 100 bar ändert, zur visuellen oder optischen Darstellung des Erreichens oder Überschreitens eines oder mehrerer Injektionsschwelldrücke eines Fluides, vorzugsweise medizinischen Fluides, wie insbesondere eines Anästhetikums, geeignet ist. Dies ist vor allem bei der Durchführung einer peripheren Regionalanästhesie von Vorteil. Bei dieser Art der Anästhesie bringt der Anästhesist die Kanülenspitze, beispielsweise durch Ultraschallkontrolle, so nah wie möglich an den betreffenden Nerven. Durch vorsichtiges Erhöhen der Kraft auf den Kolben der Spritze überprüft der Anästhesist sodann die korrekte Position der Kanülenspitze. Befindet sich die Kanülenspitze unmittelbar am oder sogar in dem Nerven, steigt der Eröffnungsinjektionsdruck des Anästhetikums in der Regel auf einen Wert von > (gesprochen: größer) 15 psi (1,034 bar) an. Der Injektionsdruck ist statisch oder im Wesentlichen statisch, da der Flüssigkeitsstrom des Anästhetikums vernachlässigbar ist. Der statische Flüssigkeitsdruck, der im gesamten Fluidpfad gleich ist, kann nun über die erfindungsgemäße Vorrichtung bzw. das piezochrome Material sichtbar gemacht werden. Dadurch erhält der Anästhesist die Möglichkeit, die Position der Kanülenspitze erforderlichenfalls zu korrigieren. Mithin kann das Risiko von Nervenschädigungen bei der Durchführung einer Regionalanästhesie signifikant reduziert werden.

Vorteilhafterweise können abhängig von der Art und der chemischen Zusammensetzung des piezochromen Materials Schwelldrücke, d.h. Druck-Schwellenwerte, deren Überschreitung beispielsweise unter medizinischen Gesichtspunkten bedenklich sein kann, sehr genau, d.h. ohne große Schwankungsbreite, und insbesondere zuverlässig sowie reproduzierbar visuell darstellen. Beispielsweise können mit Hilfe der erfindungsgemäßen Vorrichtung Injektionsschwelldrücke visualisiert werden, deren Überschreitung zu einer Schädigung von Körperzellen, insbesondere Nervenzellen, und/oder Körpergeweben, insbesondere Nervengewebe, führt.

Ein weiterer Vorteil besteht darin, dass die erfindungsgemäße medizinische Vorrichtung durch das piezochrome Material ein signifikant schnelleres Druckansprechverhalten aufweist als aus dem Stand der Technik bekannte Druckindikatoren. Durch die gegenüber gattungsgemäßen Druckindikatoren verbesserte Drucksensitivität des piezochromen Materials kann die Visualisierung und Einhaltung von insbesondere medizinisch relevanten Injektionsschwelldrücken zusätzlich optimiert werden.

Ein weiterer Vorteil der Erfindung besteht darin, dass durch das piezochrome Material auch Drücke erfasst werden können, die für medizinische Anwendungen, insbesondere im Bereich der Regionalanästhesie, vorzugsweise peripheren Regionalanästhesie, von besonderer Relevanz sind.

Ferner ist vorteilhaft, dass die erfindungsgemäße medizinische Vorrichtung gegenüber konventionellen Vorrichtungen einen signifikant einfacheren Aufbau besitzen kann. Insbesondere kann die erfindungsgemäße medizinische Vorrichtung weniger Bauteile aufweisen als aus dem Stand der Technik bekannte Vorrichtungen. Insbesondere bevorzugt kann die erfindungsgemäße Vorrichtung, abgesehen von einem etwaigen Spritzenkolben, keine beweglichen, insbesondere keine mechanisch beweglichen, und/oder keine elektronischen Bauteile aufweisen. Dadurch ist eine günstigere Fertigung der erfindungsgemäßen medizinischen Vorrichtung realisierbar. Die erfindungsgemäße medizinische Vorrichtung kann daher vorzugsweise als Einmal- oder Einwegprodukt, d.h. als ein für eine einmalige Verwendung vorgesehenes Produkt, gestaltet sein. Alternativ kann die medizinische Vorrichtung gemäß der vorliegenden Erfindung als Mehrweg-Produkt, d.h. als ein für eine mehrmalige Verwendung vorgesehenes Produkt, gestaltet sein.

Schließlich ist von Vorteil, dass die erfindungsgemäße medizinische Vorrichtung mit jeglichen Form von Konnektoren, wie beispielsweise Luer-Konnektoren, NRFit^{®}-Konnektoren und dergleichen, versehen werden kann.

In Ausgestaltung der Erfindung zeigt das piezochrome Material bei Einwirkung eines Drucks von < (gesprochen: kleiner) 50 bar, insbesondere (gesprochen: kleiner) 10 bar, bevorzugt (gesprochen: kleiner) 5 bar, besonders bevorzugt von 1 bar bis 3 bar, eine für das menschliche Auge wahrnehmbare piezochrome Farbänderung. Insbesondere die in diesem Absatz offenbarte Drucksensitivität des piezochromen Materials trägt vorteilhafterweise dazu bei, die Überschreitung eines unter medizinischen Gesichtspunkten kritischen Injektionsschwellendrucks und mithin eine Schädigung von Körperzellen und/oder Körpergewebe, insbesondere Nervenzellen und/oder Nervengewebe, zu verhindern.

In weiterer Ausgestaltung der Erfindung zeigt das piezochrome Material die für das menschliche Auge wahrnehmbare piezochrome Farbänderung innerhalb eines Zeitraumes von < (gesprochen: kleiner) 5 s, insbesondere innerhalb eines Zeitraumes von < (gesprochen: kleiner) 1 s. Insbesondere kann das piezochrome Material die für das menschliche Auge wahrnehmbare piezochrome Farbänderung innerhalb eines Zeitraumes von 0 s, insbesondere > (gesprochen: größer) 0 s, bis 2 s, bevorzugt innerhalb eines Zeitraumes von 0 s, insbesondere > (gesprochen: größer) 0 s, bis 1 s, zeigen. Dadurch ist eine quasi unmittelbare Farbänderung des piezochromen Materials bei Druckeinwirkung erzielbar. Dies trägt vorteilhafterweise ebenfalls dazu bei, das Überschreiten eines unter medizinischen Gesichtspunkten bedenklichen Injektionsschwelldruckes zu vermeiden.

Grundsätzlich kann das piezochrome Material eine reversible oder irreversible piezochrome Farbänderung zeigen. Eine reversible Farbänderung kann beispielsweise bevorzugt sein, wenn die medizinische Vorrichtung mehrmals verwendet werden soll oder das Fluid in zeitlichen Intervallen, d.h. Portionen des Fluides zeitlich nacheinander injiziert werden. Alternativ kann eine irreversible Farbänderung des piezochromen Materials bevorzugt sein. Eine solche Farbänderung des piezochromen Materials kann beispielsweise für einen Nachweis vorteilhaft sein, dass bestimmte Injektionsdrücke bei einer medizinischen Anwendung nicht überschritten wurden.

Ferner kann das piezochrome Material bei Einwirkung des Drucks einen sogenannten bathochromen Effekt, d.h. eine Rotverschiebung oder Farbvertiefung, also eine Verschiebung seines Absorptionsspektrums in den langwelligeren energieärmeren Bereich des elektromagnetischen Spektrums, zeigen. Alternativ kann das piezochrome Material bei Einwirkung des Drucks einen sogenannten hypsochromen Effekt, d.h. eine Blauverschiebung, also die Verschiebung seines Absorptionsspektrums in den kurzwelligeren, energiereicheren Bereich, des elektromagnetischen Spektrums, zeigen.

Ferner ist das piezochrome Material vorzugsweise ein sterilisierbeständiges, insbesondere ein gegenüber einer Gassterilisation, vorzugsweise einer Sterilisation mit Ethylenoxid, beständiges Material.

Bevorzugt weist das piezochrome Material ein Polymer und/oder Copolymer auf. Alternativ kann es sich bei dem piezochromen Material um ein Polymer und/oder Copolymer handeln.

Unter dem Ausdruck "Copolymer" soll im Sinne der vorliegenden Erfindung ein Polymer verstanden werden, das wenigstens zwei unterschiedliche Monomereinheiten aufweist.

In weiterer Ausgestaltung der Erfindung weist das piezochrome Material eine Matrix aus wenigstens einem Polymer und/oder Copolymer, wenigstens einen Flüssigkristall und wenigstens eine optisch aktive Substanz auf. Die wenigstens eine optisch aktive Substanz liegt vorzugsweise in der Matrix verteilt vor.

Ferner handelt es sich bei der wenigstens einen optisch aktiven Substanz vorzugsweise um eine chirale Substanz oder chiral optisch aktive Substanz.

Als Flüssigkristall kommen grundsätzlich alle bekannten Flüssigkristalle infrage.

In weiterer Ausgestaltung der Erfindung ist der wenigstens eine Flüssigkristall ausgewählt aus der Gruppe bestehend aus N-(p-Ethoxybenzyliden)-p-n-butylanilin, N-(p-Methoxybenzyliden)-p-n-butylanilin, 4-n-Alkylbenzoesäure-(4-alkylphenylester), Benzoesäurecholesterylester, Cholesterin, Tolan, Alkansäuren, Stilben, Azobenzol, 4-Phenylzimtsäure, p-Terphenyl, 1,2-Bisbenzoethylen und Mischungen von wenigstens zwei der vorgenannten Flüssigkristalle.

In weiterer Ausgestaltung der Erfindung ist die wenigstens eine optisch aktive Substanz ausgewählt aus der Gruppe bestehend aus 4-(4-Hexyloxybenzoyloxy)benzoat, 4-[[4-(Hexoyl)benzoyl]oxy]-benzoesäure-2-octylester, Cholesterylderivate und Mischungen von wenigstens zwei der vorgenannten optisch aktiven Substanzen.

In weiterer Ausgestaltung der Erfindung sind/ist das Polymer und/oder Copolymer des piezochromen Materials ein Polyacrylat, Polymethacrylat, Copolyacrylat, Copolymethacrylat oder eine Mischung von wenigstens zwei der vorgenannten Polymere und/oder Copolymere. Die Copolyacrylate und/oder Copolymethacrylate sind vorzugsweise aus wenigstens einem monofunktionalen Monomer und/oder wenigstens einem mehrfach funktionalen Vernetzer aufgebaut.

In weiterer Ausgestaltung der Erfindung ist das monofunktionale Monomer ausgewählt aus der Gruppe bestehend aus Benzylacrylat, Benzylmethacrylat, 2-Ethyl-hexylacrylat, 2-Ethylhexylmethacrylat, Methoxyethylacrylat, Methoxyethylmethacrylat, Octadecylacrylat, Octadecylmethacrylat und Mischungen von wenigstens zwei der vorgenannten Monomere.

In weiterer Ausgestaltung ist der Vernetzer ausgewählt aus der Gruppe bestehend aus 1,4-Butandioldiacrylat, 1,4-Butandioldimethacrylat, Polyethylenglykoldiacrylat, Polyethylenglykoldimethacrylat, 1,10-Decandioldiacrylat, 1,10-Decandioldimethacrylat, Bisphenol A-Glycerolat-(1-glycerol)phenoldiacrylat und Mischungen von wenigstens zwei der vorgenannten Vernetzer.

Bevorzugt weist der Vernetzer und das monfunktionale Monomer ein Gewichtsverhältnis (Vernetzer zu monofunktionalem Monomer) zwischen 0,01 und 0,8, bevorzugt zwischen 0,1 und 0,6, besonders bevorzugt zwischen 0,2 und 0,4, auf.

Ferner weist der wenigstens eine Flüssigkristall bevorzugt einen Anteil von bis zu 90 Gew.-%, insbesondere zwischen 15 Gew.-% und 60 Gew.-%, vorzugsweise zwischen 25 Gew.-% und 32 Gew.-%, bezogen auf das Gesamtgewicht des piezochromen Materials oder die Matrix des piezochromen Materials, auf.

Ferner weist die wenigstens eine optisch aktive Substanz bevorzugt einen Anteil von bis zu 10 Gew.-%, insbesondere zwischen 0,1 Gew.-% und 2,5 Gew.-%, vorzugsweise zwischen 0,15 Gew.-% und 0,55 Gew.-%, bezogen auf das Gesamtgewicht des piezochromen Materials oder der Matrix des piezochromen Materials, auf.

Bevorzugt liegt das piezochrome Material ferner in Form eines elastischen und hochgeordneten Systems vor. Vorzugsweise weist das System eine helikale Struktur auf.

Bezüglich weiterer Merkmale und Vorteile des piezochromen Materials wird auf die WO 2011/012315 A1 Bezug genommen, deren Offenbarungsgehalt in Bezug auf das dort beschriebene piezochrome Material durch ausdrückliche Bezugnahme zum Inhalt der vorliegenden Beschreibung gemacht wird.

In weiterer Ausgestaltung der Erfindung ist das piezochrome Material in einer den Fluidpfad umgebenden Wandung eines Gehäuses, d.h. in einer den Fluidpfad umgebenden Gehäusewandung, der medizinischen Vorrichtung enthalten. Das piezochrome Material kann nur in einem einzigen Wandungsabschnitt der Gehäusewandung der medizinischen Vorrichtung enthalten sein. Alternativ kann es bevorzugt sein, dass das piezochrome Material in mehreren Wandungsabschnitten der Gehäusewandung der medizinischen Vorrichtung enthalten sein. Die Wandungsabschnitte können dabei in Längsrichtung und/oder Quer- oder Umfangsrichtung der medizinischen Vorrichtung angeordnet sein. Im Falle einer medizinischen Vorrichtung mit einem zylindrischen, insbesondere kreiszylindrischen, Gehäuse können die Wandungsabschnitte ferner radial angeordnet sein. Insbesondere können sich die Wandungsabschnitte in Bezug auf das piezochrome Material voneinander unterscheiden. Dadurch können mit besonderem Vorteil unterschiedliche Injektionsdrücke, insbesondere Injektionsschwelldrücke, visuell oder optisch sichtbar gemacht werden.

In weiterer Ausgestaltung der Erfindung weist die Gehäusewandung der medizinischen Vorrichtung, vorzugsweise in Dickenrichtung der Gehäusewandung, wenigstens einen mehrschichtig, insbesondere dreischichtig, aufgebauten Wandungsabschnitt, d.h. nur einen mehrschichtig, insbesondere dreischichtig, aufgebauten Wandungsschnitt oder mehrere mehrschichtig, insbesondere dreischichtig, aufgebaute Wandungsabschnitte, auf. Bevorzugt weist eine Zwischenschicht des wenigstens einen mehrschichtig aufgebauten Wandungsabschnittes das piezochrome Material auf oder besteht aus dem piezochromen Material. Insbesondere kann die Zwischenschicht, insbesondere in Längs- oder Umfangsrichtung des Gehäuses der medizinischen Vorrichtung, aus mehreren, vorzugsweise unmittelbar, nebeneinander angeordneten Zwischenschichtabschnitten zusammengesetzt sein. Vorzugsweise unterscheiden sich die Zwischenschichtabschnitte in Bezug auf das piezochrome Material voneinander. Dadurch lassen sich mit besonderem Vorteil unterschiedliche Injektionsdrücke optisch sichtbar machen. Bevorzugt weist die Zwischenschicht eine Schichtdicke von 0 mm, insbesondere > (gesprochen: größer) 0 mm, bis 1 mm, insbesondere 0 mm, insbesondere > (gesprochen: größer) 0 mm, bis 0,6 mm, vorzugsweise 0 mm, insbesondere > (gesprochen: größer) 0 mm, bis 0,2 mm, auf.

In weiterer Ausgestaltung der Erfindung weist der wenigstens eine mehrschichtig, insbesondere dreischichtig, aufgebaute Wandungsabschnitt ferner eine Innenschicht auf, die die Zwischenschicht innenseitig, d.h. auf einer dem Fluidpfad zugewandten Seite, bedeckt, vorzugsweise unmittelbar und insbesondere vollständig. Besonders bevorzugt steht die Innenschicht in unmittelbaren Kontakt zu dem Fluidpfad. Die Innenschicht weist vorzugsweise ein biokompatibles, d.h. medizinisch verträgliches, Material auf oder besteht vorzugsweise aus einem solchen Material. Dadurch ist vorteilhafterweise ein Schutz des piezochromen Materials vor einem unmittelbaren Kontakt mit dem Fluid und mithin die Vermeidung einer etwaigen Beeinträchtigung des piezochromen Materials durch das Fluid oder Bestandteile davon erzielbar. Bevorzugt weist die Innenschicht eine Schichtdicke von 0 mm, insbesondere > (gesprochen: größer) 0 mm, bis 1 mm, insbesondere 0 mm, insbesondere > (gesprochen: größer) 0 mm, bis 0,3 mm, vorzugsweise 0 mm, insbesondere > (gesprochen: größer) 0 mm, bis 0,1 mm, auf. Durch eine solche Schichtdicke ist vorteilhafterweise gewährleistet, dass der vom Fluid ausgeübte Druck nahezu unmittelbar auf das piezochrome Material übertragen und somit die Farbänderung des piezochromen Materials zuverlässig und reproduzierbar herbeigeführt werden kann.

In weiterer Ausgestaltung der Erfindung ist das biokompatible Material ausgewählt aus der Gruppe bestehend aus Polyethylenterephthalat (PET), Polyetheretherketon (PEEK), Polypropylen (PP), Polyphenylsulfon (PPSU), Polyoxymethylen (POM) und Mischungen von wenigstens zwei der vorgenannten biokompatiblen Materialien.

In weiterer Ausgestaltung der Erfindung weist der wenigstens eine mehrschichtig, insbesondere dreischichtig, aufgebaute Wandungsabschnitt ferner eine Außenschicht auf, die die Zwischenschicht außenseitig, d.h. auf einer dem Fluidpfad abgewandten Seite, bedeckt, vorzugsweise unmittelbar und insbesondere vollständig. Besonders bevorzugt bildet die Außenschicht einen Teil der Außenseite der medizinischen Vorrichtung, insbesondere des Gehäuses der medizinischen Vorrichtung. Vorzugsweise weist die Außenschicht ein transparentes, d.h. lichtdurchlässiges, Material auf. Alternativ kann die Außenschicht bevorzugt aus einem solchen Material bestehen. Dadurch ist vorteilhafterweise eine visuelle oder optische Darstellung der Farbänderung des piezochromen Materials erzielbar. Die Außenschicht kann dabei insbesondere als Bildschirm oder Display gestaltet sein.

Unter dem Ausdruck "transparentes Material" oder "lichtdurchlässiges Material" soll im Sinne der vorliegenden Erfindung ein Material verstanden werden, das für Licht mit einer Wellenlänge von 380 nm bis 780 nm durchlässig ist.

In weiterer Ausgestaltung der Erfindung ist das transparente Material ausgewählt aus der Gruppe bestehend aus Polycarbonat (PC), Polymethylmethacrylat (PMMA), Polyurethan (PU) und Mischungen von wenigstens zwei der vorgenannten transparenten Materialien.

Besonders bevorzugt ist der wenigstens eine mehrschichtig, insbesondere dreischichtig, aufgebaute Wandungsabschnitt der Gehäusewandung der medizinischen Vorrichtung innen nach außen aus einer Innenschicht, Zwischenschicht und Außenschicht, wie in den vorherigen Absätzen beschrieben, aufgebaut.

Wie bereits erwähnt, ist das piezochrome Material vorzugsweise in einer Wandung eines Gehäuses der medizinischen Vorrichtung enthalten. Das Gehäuse kann grundsätzlich jede denkbare Form besitzen, die dazu geeignet ist, einen Fluidpfad zu bilden oder zu definieren, durch den das Fluid befördert werden kann. Beispielsweise kann das Gehäuse eine prismatische, insbesondere würfel- oder quaderförmige, Form oder eine zylindrische, insbesondere kreiszylindrische, Form besitzen.

Die medizinische Vorrichtung, insbesondere das Gehäuse der medizinischen Vorrichtung, weist, vorzugsweise an einem distalen Ende der medizinischen Vorrichtung, insbesondere des Gehäuses der medizinischen Vorrichtung, eine erste Konnektoreinrichtung zum Verbinden der medizinischen Vorrichtung, insbesondere des Gehäuses der medizinischen Vorrichtung, mit einem Injektionsschlauch auf. Unter dem Ausdruck "distales Ende" soll hierbei das Ende der medizinischen Vorrichtung, insbesondere des Gehäuses der medizinischen Vorrichtung, verstanden werden, dass entfernt zur Körpermitte eines Anwenders, insbesondere Anästhesisten, gelegen ist.

Weiter weist die medizinische Vorrichtung, insbesondere das Gehäuse der medizinischen Vorrichtung, vorzugsweise an einem proximalen Ende der medizinischen Vorrichtung, insbesondere des Gehäuses der medizinischen Vorrichtung, eine zweite Konnektoreinrichtung zum Verbinden der medizinischen Vorrichtung, insbesondere des Gehäuses der medizinischen Vorrichtung, mit einem Spritzenkörper auf. Unter dem Ausdruck "proximales Ende" soll hierbei das Ende der medizinischen Vorrichtung, insbesondere des Gehäuses der medizinischen Vorrichtung, verstanden werden, dass zur Körpermitte hin eines Anwenders, insbesondere Anästhesisten, gelegen ist.

Die in den vorherigen Abschnitten beschriebenen Konnektoreinrichtungen können beispielsweise als Luer-Lock-Anschlüsse, insbesondere als männliche Luer-Lock-Anschlüsse, weibliche Luer-Lock-Anschlüsse, männlicher Luer-Lock Anschluss und weiblicher Luer-Lock-Anschluss oder als NRFit^{®}-Konnektoren gestaltet sein.

Ferner ist die medizinische Vorrichtung vorzugsweise sterilisierbeständig, insbesondere beständig gegenüber einer Gassterilisation, vorzugswiese einer Sterilisation mit Ethylenoxid. Weitere Merkmale und Vorteile der Erfindung ergeben sich aus den Ansprüchen sowie aus der nachfolgenden Beschreibung bevorzugter Ausführungsformen anhand von Figuren sowie von Beispielen. Die nachfolgend beschriebenen Ausführungsformen dienen der weiteren Erläuterung der Erfindung.

### KURZBESCHREIBUNG DER ZEICHNUNGEN

In den Figuren ist Folgendes schematisch gezeigt:
- Fig. 1:: eine Ausführungsform einer erfindungsgemäßen medizinischen Vorrichtung,
- Fig. 2:: eine Querschnittsdarstellung einer weiteren Ausführungsform einer erfindungsgemäßen medizinischen Vorrichtung,
- Fig. 3:: eine Draufsicht auf die in Fig. 2 dargestellte Ausführungsform einer erfindungsgemäßen medizinischen Vorrichtung,
- Fig. 4:: eine Detailansicht einer Gehäusewandung einer weiteren Ausführungsform einer erfindungsgemäßen medizinischen Vorrichtung,
- Fig. 5:: eine Draufsicht auf eine Gehäusewandung einer weiteren Ausführungsform einer erfindungsgemäßen medizinischen Vorrichtung,
- Fig. 6:: eine Ausführungsform einer nicht erfindungsgemäßen medizinischen Vorrichtung,
- Fig. 7:: eine weitere Ausführungsform einer nicht erfindungsgemäßen medizinischen Vorrichtung,
- Fig. 8:: eine weitere Ausführungsform einer nicht erfindungsgemäßen medizinischen Vorrichtung und
- Fig. 9: eine weitere Ausführungsform einer nicht erfindungsgemäßen medizinischen Vorrichtung.

### DETAILLIERTE BESCHREIBUNG DER AUSFÜHRUNGSBEISPIELE

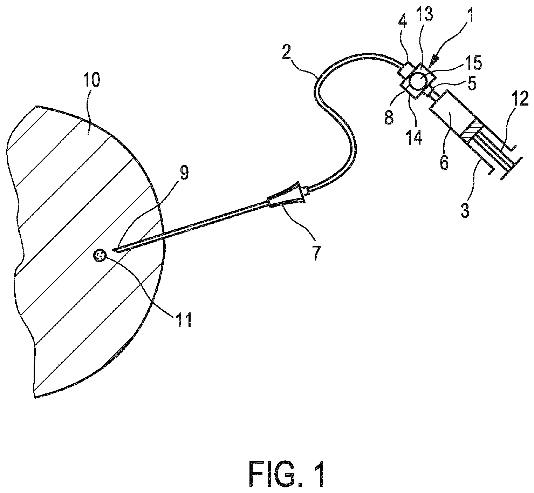

Fig. 1 zeigt schematisch eine Ausführungsform einer erfindungsgemäßen medizinischen Vorrichtung 1 zum visuellen oder optischen Darstellen eines Injektionsdruckes, insbesondere Injektionsschwelldruckes, eines Anästhetikums bei der Durchführung einer peripheren Regionalanästhesie.

Die medizinische Vorrichtung 1 ist zwischen einem Injektionsschlauch 2 und einer Spritze 3 geschaltet. Hierzu weist die medizinische Vorrichtung vorteilhafterweise eine Konnektoreinrichtung 4 zum Verbinden der medizinischen Vorrichtung 1 mit dem Injektionsschlauch 2 sowie eine weitere Konnektoreinrichtung 5 zum Verbinden der medizinischen Vorrichtung 1 mit der Spritze 3 auf. Die Konnektoreinrichtungen 4 und 5 können beispielsweise jeweils als Luer-Lock-Anschlüsse gestaltet sein.

Die Spritze 3 weist einen Hohlraum (Lumen) 6 auf, der mit einem Anästhetikum befüllt ist. An einem distalen Ende, d.h. an einem entfernt zur Körpermitte eines Anwenders, vorliegend eines Anästhesisten, gelegenen Ende, des Injektionsschlauchs 2 ist eine Kanüle 7 befestigt.

Die medizinische Vorrichtung 1 weist einen Fluidpfad 8 für das Anästhetikum sowie ein piezochromes Material auf. Das piezochrome Material zeigt vorzugsweise bei Einwirkung eines Drucks von < 10 bar, bevorzugt < 5 bar, besonders bevorzugt von 1 bar bis 3 bar, eine für das menschliche Auge wahrnehmbare piezochrome Farbänderung. Bei dem piezochromen Material kann es sich insbesondere um einen piezochromen Kunststoff oder um eine piezochrome Zusammensetzung handeln. Vorzugsweise weist das piezochrome Material eine Matrix aus wenigstens einem Polymer und/oder Copolymer, wenigstens einen Flüssigkristall und wenigstens eine optisch aktive Substanz, insbesondere chiral optisch aktive Substanz, auf. Die optisch aktive Substanz liegt vorzugsweise in der Matrix verteilt vor.

Das vorgenannte Polymer kann beispielsweise ein vernetztes Polymethacrylat sein. Bei dem vorgenannten Copolymer kann es sich beispielsweise um ein vernetztes Copolymethacrylat handeln. Bei dem wenigstens einen Flüssigkristall kann es sich beispielsweise um eine Flüssigkristallmischung aus vier verschiedenen Alkylcyanobiphenylderivaten und einem Alkoxycyanobiphenylderivat handeln. Bei der optisch aktiven Substanz kann es sich beispielsweise um die Substanz S-811, also 4-(4-Hexyloxybenzoyloxy)-benzoat, Cholesterylderivate und/oder Mischungen hieraus, handeln.

Zum Durchführen der peripheren Regionalanästhesie wird die Spitze 9 der Kanüle 7, beispielsweise unter Ultraschallkontrolle, so nahe wie möglich an einen in einem peripheren Gewebebereich 10 gelegenen Nerv 11 gebracht. Der Nerv 11 ist in der Fig. 1 im Querschnitt dargestellt. Dabei überprüft der Anästhesist die korrekte Position der Kanülenspitze 9 durch vorsichtiges Erhöhen der Kraft auf einen Kolben 12 der Spritze 3. Befindet sich die Spitze 9 der Kanüle 7 unmittelbar am oder sogar im Nerv 11, steigt der Eröffnungsinjektionsdruck, der im Prinzip statisch ist, auf einen definierten Wert, in der Regel auf einen Wert größer 1,034 bar (> 15 psi), an. Der statische Druck des Anästhetikums, der im gesamten Fluidpfad 8 der medizinischen Vorrichtung 1 gleich ist, kann nun über das piezochrome Material der medizinische Vorrichtung 1 optisch sichtbar gemacht werden. Dies wird dadurch erreicht, dass der Fluidpfad 8, der durch die medizinische Vorrichtung 1 verläuft, an einer Gehäusewandung 13, d.h. an einer Wandung 13 eines Gehäuses 14 der medizinischen Vorrichtung 1 vorbeigeführt wird, wobei die Gehäusewandung 13 in ihrer Dickenrichtung wenigstens einen mehrschichtig, insbesondere dreischichtig, aufgebauten Wandungsabschnitt 15 mit dem piezochromen Material aufweist. Bevorzugt weist der Wandungsabschnitt 15 eine Zwischenschicht auf, die auf einer dem Fluidpfad 8 zugewandten Seite mit einer Innenschicht und auf einer dem Fluidpfad 8 abgewandten Seite mit einer Außenschicht beschichtet ist. Bevorzugt weist die Zwischenschicht das piezochrome Material auf oder besteht aus dem piezochromen Material. Die Innenschicht weist vorzugsweise ein biokompatibles Material, vorzugsweise zum Schutz des piezochromen Materials vor dem Fluid, auf oder besteht aus einem solchen Material. Bei dem biokompatiblen Material kann es sich beispielsweise um Polyethylenterephthalat, Polyetheretherketon, Polypropylen, Polyphenylsulfon oder Polyoxymethylen handeln. Die Außenschicht weist vorzugsweise ein transparentes Material auf oder besteht aus einem solchen Material. Das transparente Material kann beispielsweise Polycarbonat, Polymethylmethacrylat oder Polyurethan sein. Zweckmäßigerweise steht die Innenschicht in direktem Kontakt mit dem Fluidpfad 8 der medizinischen Vorrichtung 1, wohingegen die Außenschicht zweckmäßigerweise einen Teil der Außenseite des Gehäuses 14 der medizinischen Vorrichtung 1 bildet. Dabei kann die Außenschicht insbesondere als Bildschirm oder Display gestaltet sein.

Bei Erhöhung des Injektionsdruckes auf einen definierten und von der Zusammensetzung des piezochromen Materials abhängigen Wert ändert das piezochrome Material seine Farbe. Die Farbänderung kann reversibel oder irreversibel erfolgen. Vorteilhafterweise lässt sich der Schwelldruck des Injektionsdruckes über die Zusammensetzung des piezochromen Materials sehr genau und insbesondere reproduzierbar einstellen. Auf diese Weise kann das Erreichen oder Überschreiten eines definierten Druckes optisch sichtbar gemacht werden. Aufgrund der oben erwähnten transparenten Außenschicht ist die Farbänderung des piezochromen Materials nach außen hin sichtbar. Dadurch erkennt der Anästhesist, dass er die Kanülenspitze 9 entweder zu nahe oder sogar in den zu blockierenden Nerven positioniert hat, und kann die Position der Kanülenspitze 9 entsprechend korrigieren. Dadurch kann mit besonderem Vorteil eine Schädigung des Nervs 11 vermieden werden.

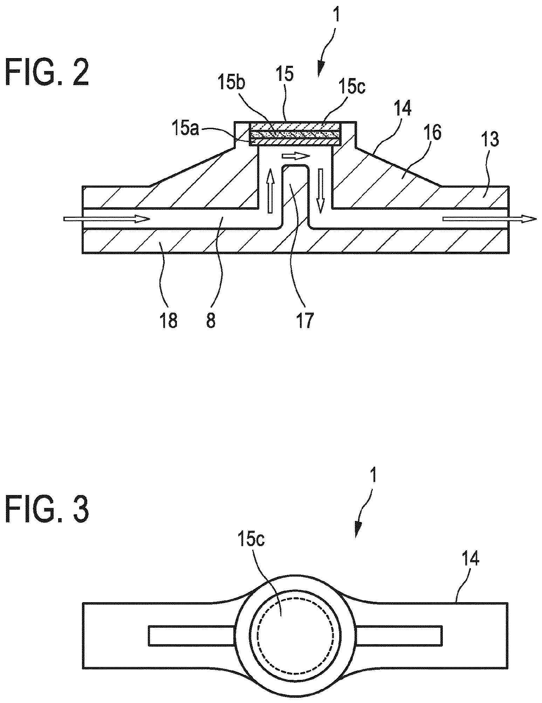

Fig. 2 zeigt schematisch eine Querschnittsansicht einer weiteren Ausführungsform einer erfindungsgemäßen medizinischen Vorrichtung 1 zum visuellen oder optischen Darstellen eines Injektionsdruckes, insbesondere Injektionsschwelldruckes, eines Fluides, wie beispielsweise eines Anästhetikums oder einer anästhetikumhaltigen Flüssigkeit.

Die medizinische Vorrichtung 1 weist einen Fluidpfad 8 auf, der von einer Gehäusewandung 13, d.h. einer Wandung 13 eines Gehäuses 14, der medizinischen Vorrichtung 1 umgeben wird. Die Wandung 11 weist einen Wandungsabschnitt 15 auf. Der Wandungsabschnitt 15 besitzt einen dreischichtigen Aufbau aus einer einer Innenschicht 15a, Zwischenschicht 15b und einer Außenschicht 15c. Die Zwischenschicht 15b weist ein piezochromes Material auf oder besteht aus einem solchen Material. Die Innenschicht 15a weist ein biokompatibles Material zum Schutz des piezochromen Materials vor einem unmittelbaren Kontakt mit dem Fluid auf oder besteht aus einem solchen Material. Die Außenschicht 15c weist ein transparentes Material auf oder besteht aus einem solchen Material. Bezüglich weiterer Merkmale und Vorteile des piezochromen Materials, biokompatiblen Materials sowie transparenten Materials wird auf die bisherige Beschreibung, insbesondere auf die in der Figurenbeschreibung zur Fig. 1 erwähnten Merkmale und Vorteile Bezug genommen.

Die Innenschicht 15a bedeckt die Zwischenschicht 15b, vorzugsweise unmittelbar, auf einer dem Fluidpfad 8 zugewandten Seite. Dabei steht die Innenschicht 15a vorzugsweise in unmittelbarem Kontakt mit dem Fluidpfad 8. Dagegen bedeckt die Außenschicht 15c die Zwischenschicht 15b, vorzugsweise unmittelbar, auf einer dem Fluidpfad 8 abgewandten Seite. Bevorzugt bildet die Außenschicht 15c einen Teil der Außenseite des Gehäuses 14 der medizinischen Vorrichtung 1.

Das Gehäuse 14 der medizinischen Vorrichtung 1 kann ferner, insbesondere im Bereich des Wandungsabschnitts 15, eine Verstärkung, insbesondere eine Verstärkungsrippe, 16 aufweisen. Ferner kann die medizinische Vorrichtung 1 einen innenseitig in Richtung des Wandungsabschnitts 15 ausgebildeten Steg 17 aufweisen. Bevorzugt ist der Steg 17 Teil eines dem Wandungsabschnitt 15 gegenüberliegend angeordneten Teils 18 des Gehäuses 14. Durch den Steg 17 kann vorteilhafterweise die Bildung von Luftblasen beim Injizieren des Fluides unterbunden und der Fluidstrom direkt am Wandungsabschnitt 15 und mithin an dem darin befindlichen piezochromen Material vorbeigeführt werden. Ferner ist hierdurch vorteilhafterweise eine Reduzierung des Totraumvolumens realisierbar.

Zur weitergehenden Funktionsweise der in Fig. 2 schematisch gezeigten medizinischen Vorrichtung 1 wird vollständig auf die bisherige Beschreibung, insbesondere auf die Figurenbeschreibung zur Fig. 1, Bezug genommen. Die dort insoweit gemachten Ausführungen gelten sinngemäß.

Fig. 3 zeigt schematisch eine Draufsicht auf die in Fig. 2 dargestellte Ausführungsform einer erfindungsgemäßen medizinischen Vorrichtung 1.

Über die an der Außenseite des Gehäuses 14 befindliche transparente Außenschicht 15c wird eine Farbänderung des piezochromen Materials und mithin das Erreichen oder Überschreiten eines Injektionschwelldruckes beim Injizieren des Fluides optisch erkennbar. Gegebenenfalls kann ein Anästhesist, insbesondere bei der Durchführung eines peripheren Regionalanästhesieverfahrens, die Position einer Kanülenspitze korrigieren.

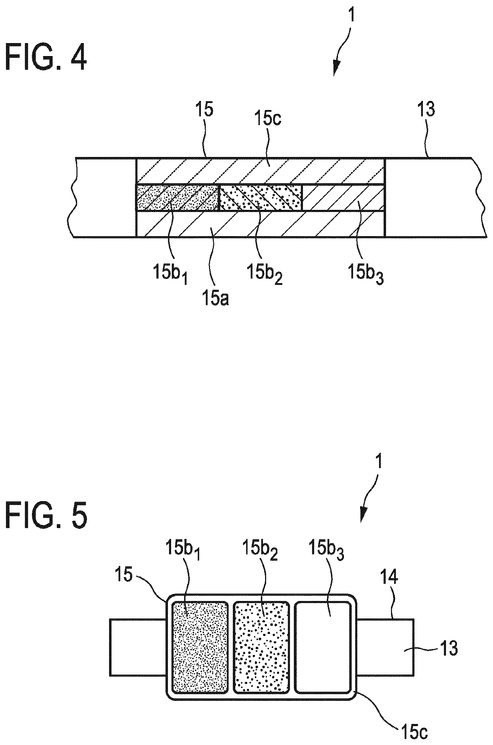

Fig. 4 zeigt schematisch die Detailansicht einer Gehäusewandung 13 einer weiteren Ausführungsform einer erfindungsgemäßen medizinischen Vorrichtung zum visuellen oder optischen Darstellen eines Injektionsdruckes, insbesondere Injektionsschwelldruckes, eines Fluides, wie beispielsweise eines Anästhetikums oder einer anästhetikumhaltigen Flüssigkeit.

Die Gehäusewandung 13 weist einen Wandungsabschnitt 15. Dieser besitzt in Dickenrichtung der Gehäusewandung 13 einen dreischichtigen Aufbau aus einer Innenschicht 15a, einer Zwischenschicht 15b sowie einer Außensicht 15c. Die Zwischenschicht 15b besteht aus mehreren Zwischenschichtabschnitten, die in Längsrichtung der Gehäusewandung, vorzugsweise unmittelbar, nebeneinander angeordnet sind. Vorzugsweise weisen die Zwischenschichtabschnitte jeweils ein unterschiedliches piezochromes Material auf oder bestehen jeweils aus einem unterschiedlichen piezochromen Material. Dadurch können vorteilhafterweise unterschiedliche Injektionsschwelldrücke visualisiert werden. Beispielsweise kann die Zwischenschicht 15b, wie in Fig. 4 dargestellt, aus drei derartiger Zwischenschichtabschnitten 15b_{1,} 15b₂ und 15b₃ bestehen. Beispielsweise können die piezochromen Materialien für die Zwischenschichtabschnitte 15b_{1,} 15b₂ und 15b₃ derart gewählt werden, dass das piezochrome Material des Zwischenschichtabschnittes 15b₁ eine Farbänderung bei Einwirkung eines Drucks ≥ 0,69 bar (≥ 10 psi), das piezochrome Material des Zwischenschichtabschnittes 15b₂ eine Farbänderung bei Einwirkung eines Drucks ≥ 1,03 bar (≥ 15 psi) und das piezochrome Material des Zwischenschichtabschnittes 15b₃ eine Farbänderung bei einer Einwirkung eines Druckes ≥ 1,38 bar (≥ 20 psi) zeigt. Bezüglich weiterer geeigneter piezochromer Materialien wird auf die bereits in der bisherigen Beschreibung, insbesondere in der Figurenbeschreibung zur Fig. 1, erwähnten piezochromen Materialien Bezug genommen.

Auf einer einem Fluidpfad zugewandten Seite wird die Zwischenschicht 15b von der Innenschicht 15a bedeckt, vorzugsweise unmittelbar und vollständig. Vorzugsweise steht die Innenschicht 15a dabei in unmittelbarem Kontakt mit dem Fluidpfad. Die Innenschicht 15a weist ein biokompatibles Material zum Schutz der piezochromen Materialien der Zwischenschicht 15b vor einem unmittelbaren Kontakt mit einem Fluid, das durch den Fluidpfad befördert wird, auf oder besteht aus einem solchen Material. Bezüglich geeigneter biokompatibler Materialien wird auf die bereits in der bisherigen Beschreibung, insbesondere in der Figurenbeschreibung zur Fig. 1, erwähnten biokompatiblen Materialien Bezug genommen.

Die Außenschicht 15c bedeckt die Zwischenschicht 15b vorzugsweise auf einer einem Fluidpfad abgewandten Seite, vorzugsweise unmittelbar und vollständig. Bevorzugt bildet die Außenschicht 15c dabei einen Teil der Außenseite des Gehäuses 14 der medizinischen Vorrichtung 1. Die Innenschicht 15c weist ein transparentes Material auf oder besteht aus einem solchen Material. Dadurch wird eine Farbänderung der piezochromen Materialien für einen Anwender, insbesondere Anästhesisten, optisch erkennbar. Dabei kann die Außenschicht insbesondere als Bildschirm oder Display gestaltet sein.

Bezüglich geeigneter transparenter Materialien wird auf die bereits in der bisherigen Beschreibung, insbesondere in der Figurenbeschreibung zur Fig. 1, erwähnten transparenten Materialien Bezug genommen.

Fig. 5 zeigt schematisch eine Draufsicht auf eine Gehäusewandung einer weiteren Ausführungsform einer erfindungsgemäßen medizinischen Vorrichtung 1.

Die medizinische Vorrichtung 1 weist ein Gehäuse 14 mit einer Gehäusewandung 13 auf. Die Gehäusewandung 13 weist einen Wandungsabschnitt 15 auf, der in Dickenrichtung der Gehäusewandung gegenüber der übrigen Gehäusewandung verbreitert ist. Der Wandungsabschnitt 15 besitzt den in der Figurenbeschreibung zur Fig. 4 beschriebenen Aufbau. Über die Außenschicht 15c, die insbesondere als Bildschirm oder Display gestaltet sein kann, können die durch die piezochromen Materialien der Zwischenschichtabschnitte 15b_{1,} 15b₂ und 15b₃ bei Einwirkung unterschiedlicher Drücke hervorgerufenen Farbänderungen visualisiert werden.

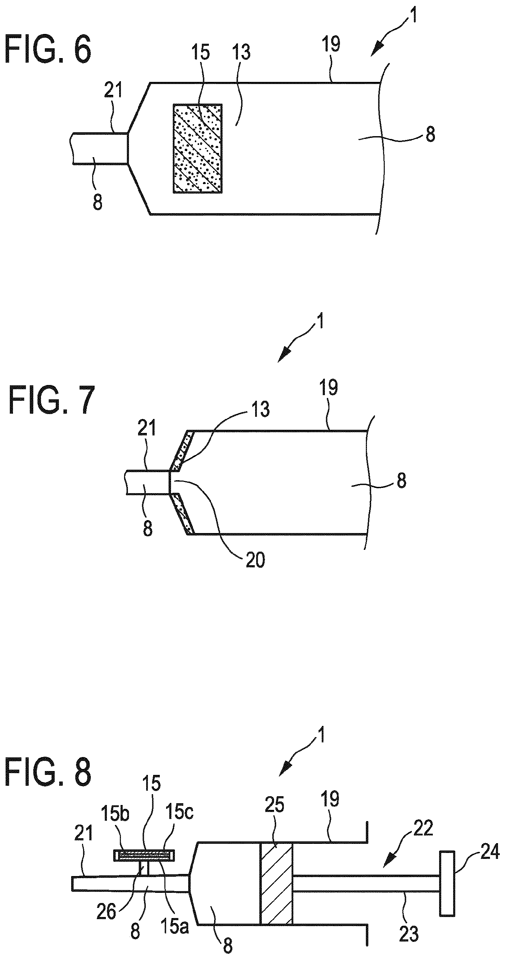

Fig. 6 zeigt schematisch eine Ausführungsform einer nicht erfindungsgemäßen medizinischen Vorrichtung 1 zum visuellen oder optischen Darstellen eines Injektionsdruckes, insbesondere Injektionsschwelldruckes, eines Fluides, wie beispielsweise eines Anästhetikums oder einer anästhetikumhaltigen Flüssigkeit.

Die medizinische Vorrichtung 1 ist als Spritze mit einem Spritzenkörper 19 mit einer vorzugsweise konisch oder zylindrisch geformten Auslassdüse 21 gestaltet. Der Spritzenkörper 19 ist vorzugsweise zylindrisch, insbesondere kreiszylindrisch, gestaltet. Die medizinische Vorrichtung 1 weist einen Fluidpfad 8 auf, der sowohl vom Hohlraum des Spritzenkörpers 19 als auch vom Hohlraum der Auslassdüse 21 gebildet wird.

Weiter weist der Spritzenkörper 19 eine Mantelwandung 13 mit einem Wandungsabschnitt 15 auf. Der Wandungsabschnitt 15 besitzt in Dickenrichtung der Mantelwandung einen dreischichtigen Aufbau aus einer Innenschicht, Zwischenschicht sowie Außenschicht. Die Innenschicht bedeckt die Zwischenschicht auf einer dem Fluidpfad 8 zugewandten Seite, während die Außenschicht die Zwischenschicht auf einer dem Fluidpfad 8 abgewandten Seite bedeckt. Bevorzugt steht die Innenschicht in unmittelbarem Kontakt mit dem Fluidpfad 8. Bevorzugt bildet die Außenschicht einen Teil der Außenseite des Spritzenkörpers 19. Die Zwischenschicht weist ein piezochromes Material auf oder besteht aus einem piezochromen Material. Die Innenschicht weist ein biokompatibles Material zum Schutz des piezochromen Materials von einem unmittelbaren Kontakt mit dem Fluid auf oder besteht aus einem solchen biokompatiblen Material. Die Außenschicht weist ein transparentes Material auf oder besteht aus einem transparenten Material. Bezüglich geeigneter piezochromer Materialien, biokompatibler Materialien sowie transparenter Materialien sowie der Funktionsweise der medizinischen Vorrichtung 1 wird auf die bisherige Beschreibung, insbesondere auf die Figurenbeschreibung zur Fig. 1, Bezug genommen.

Fig. 7 zeigt schematisch eine weitere Ausführungsform einer nicht erfindungsgemäßen medizinischen Vorrichtung 1 zum visuellen oder optischen Darstellen eines Injektionsdruckes, insbesondere Injektionsschwelldruckes, eines Fluides, wie beispielsweise eines Anästhetikums oder einer anästhetikumhaltigen Flüssigkeit.

Die medizinische Vorrichtung 1 ist (ebenfalls) als Spritze mit einem Spritzenkörper 19 gestaltet. Der Spritzenkörper 19 ist vorzugsweise zylindrisch, insbesondere kreiszylindrisch, gestaltet. Die medizinische Vorrichtung 1 weist einen Fluidpfad 8 auf, der sowohl vom Hohlraum des Spritzenkörpers 19 als auch vom Hohlraum der Auslassdüse 21 gebildet wird.

Weiter weist der Spritzenkörper 19 eine Stirnwandung 13 mit einem dreischichtigen Aufbau aus einer Innenschicht, Zwischenschicht und Außenschicht auf. Die Stirnwandung umgibt eine Austrittsöffnung 20, die in eine konisch oder zylindrisch geformte Auslassdüse 21 des Spritzenkörpers 19 mündet.

Bezüglich weiterer Merkmale und Vorteile sowie der weiteren Funktionsweise der in Fig. 7 gezeigten medizinischen Vorrichtung, insbesondere bezüglich der vorgenannten Zwischenschicht, Innenschicht und Außenschicht, wird vollständig auf die bisherige Beschreibung, insbesondere auf die Figurenbeschreibungen zu den Fig. 1 und 6, Bezug genommen.

Fig. 8 zeigt eine weitere Ausführungsform einer nicht erfindungsgemäßen medizinischen Vorrichtung 1 zum visuellen oder optischen Darstellen eines Injektionsdruckes, insbesondere Injektionsschwelldruckes, eines Fluides, wie beispielsweise eines Anästhetikums oder einer anästhetikumhaltigen Flüssigkeit.

Die medizinische Vorrichtung 1 ist (ebenfalls) als Spritze gestaltet und weist einen Spritzenkörper 19 mit einer vorzugsweise konisch oder zylindrisch gestalteten Auslassdüse 21 und einen Spritzenkolben 22 auf. Der Spritzenkörper 19 ist vorzugsweise zylindrisch, insbesondere kreiszylindrisch, gestaltet. Der Spritzenkolben 22 weist eine Stange 23, einen Kopf 24 sowie einen Stempel 25 auf. Die medizinische Vorrichtung 1 weist einen Fluidpfad 8 auf, der sowohl vom Hohlraum des Spritzenkörpers 19 als auch vom Hohlraum der Auslassdüse 21 gebildet wird.

Die Auslassdüse 21 besitzt eine Ab- oder Auszweigung 26 mit einem geschlossenen Ende 15. Das geschlossene Ende 15 bildet somit einen ab- oder ausgezweigten Wandungsabschnitt des Spritzenkörpers 19.

Bevorzugt weist das geschlossene Ende bzw. der ab- bzw. ausgezweigte Wandungsabschnitt 15 einen dreischichtigen Aufbau aus einer Innenschicht 15a, Zwischenschicht 15b und Außenschicht 15c auf.

Bezüglich weiterer Merkmale und Vorteile sowie der weiteren Funktionsweise der in Fig. 8 gezeigten medizinischen Vorrichtung, insbesondere bezüglich der vorgenannten Zwischenschicht, Innenschicht und Außenschicht, wird vollständig auf die bisherige Beschreibung, insbesondere auf die Figurenbeschreibungen zu den Fig. 1 und 6, Bezug genommen.

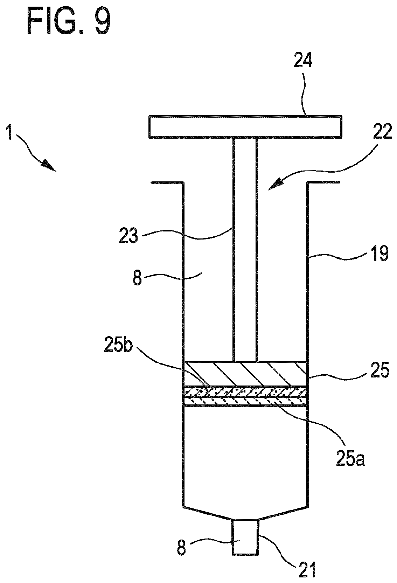

Fig. 9 zeigt eine weitere Ausführungsform einer nicht erfindungsgemäßen medizinischen Vorrichtung 1 zum visuellen oder optischen Darstellen eines Injektionsdruckes, insbesondere Injektionsschwelldruckes, eines Fluides, wie beispielsweise eines Anästhetikums oder einer anästhetikumhaltigen Flüssigkeit.

Die medizinische Vorrichtung 1 ist (ebenfalls) als Spritze gestaltet und besitzt einen Spritzenkörper 19 mit einer vorzugsweise konusförmigen oder zylindrisch gestalteten Auslassdüse 21 sowie einen Spritzenkolben 22 mit einer Stange 23, einem Kopf 24 und einem Stempel 25. Die medizinische Vorrichtung 1 weist einen Fluidpfad 8 auf, der sowohl vom Hohlraum des Spritzenkörpers 19 als auch vom Hohlraum der Auslassdüse 21 gebildet wird.

Der Stempel 25 des Spritzenkolbens 22 weist eine Schicht 25b auf, die von einer Schicht 25a bedeckt wird, wobei die Schicht 25a einen Teil der Außenseite des Stempels 25 bildet. Die Schicht 25b weist ein piezochromes Material auf oder besteht aus einem solchen Material. Die Schicht 25a weist ein transparentes Material auf oder besteht aus einem transparenten Material. Auf diese Weise kann eine Farbänderung des piezochromen Materials für einen Anwender, insbesondere Arzt, visualisiert werden.

Bezüglich weiterer Merkmale und Vorteile sowie der weiteren Funktionsweise der in Fig. 8 gezeigten medizinischen Vorrichtung, insbesondere bezüglich des vorgenannten piezochromen Materials sowie des vorgenannten transparenten Materials, wird vollständig auf die bisherige Beschreibung, insbesondere auf die Figurenbeschreibungen zu den Fig. 1 und 6, Bezug genommen.

### BEISPIELTEIL

### 1. Herstellung eines piezochromen Materials gemäß der vorliegenden Erfindung (einschließlich einer entsprechend aufgebauten Folie)

### 1.1 Herstellung einer Mischung aus einem Flüssigkristall und einer chiral optisch aktiven Substanz

Zu einer Flüssigkristallmischung E5 (Mischung bestehend aus vier Komponenten Alkylcyanobiphenylderivaten und einer Komponente Alkoxycyanobiphenylderivat) wurde die optisch aktive Substanz S-811 mit einem Gewichtsanteil von 22 % hinzugemischt. Die Mischung wurde über den Klärpunkt der E5-Komponente auf 58 °C erwärmt und anschließend bei Raumtemperatur wieder abgekühlt.

### 1.2 Herstellung einer Mischung aus einem monofunktionalen Methacrylat-Monomer und einem Vernetzer

Zu dem Monomerbenzylmethacrylat wurden 34 Gew.-% DDA dotiert. Anschließend wurde die Mischung während 25 min auf 60 °C erwärmt.

Die gemäß 1.1 und 1.2 hergestellten Mischungen wurden in einem Verhältnis von 1:2,4 bei einer Temperatur zwischen 50 °C und 55 °C gemischt. Abschließend wurden 0,2 Gew.-% Polymerisationsinitiator Genocure LTM hinzudotiert. Das piezochrome Material wurde mit einer Schichtdicke von 25 µm zwischen zwei jeweils 15 µm dicken Polypropylenfolien, die mit Polyamid beschichtet waren (kleiner 1 µm), montiert und während 15 min unter UV-Licht polymerisiert. Im Ergebnis wurde eine rot reflektierende Folie erhalten, die leicht beweglich und flexibel war und bei Druckerhöhung von 0,4 bar zu einer für das Auge grün reflektierenden Folie wurde. Wurde der Druck nochmals um 0,7 bar erhöht, wurde die Farbe blau. Der Prozess war reversibel. Die Farbänderung der Folie erfolgte in weniger als 1 s.

### 2. Herstellung eines weiteren piezochromen Materials gemäß der vorliegenden Erfindung (einschließlich einer entsprechend aufgebauten Folie)

### 2.1 Herstellung einer Mischung aus einem Flüssigkristall und einer chiral optisch aktiven Substanz

Zu dem Flüssigkristall EBBA wurde die optisch aktive Substanz Cholesteryloleylcarbonat mit 27 Gew.-% dotiert. Die Mischung wurde über den Klärpunkt von EBBA auf 52 °C erwärmt.

### 2.2 Herstellung einer Mischung aus einem monofunktionalen Acrylatmonomer und einem Vernetzer

Zu dem Monomer Octadilacrylat wurden 28 Gew.-% des Vernetzers Polyethylenglykoldiacrylat, M70 dotiert, wobei die Mischung auf 55 °C erwärmt wurde.

Die gemäß 2.1 und 2.2 hergestellten Mischungen wurden im Verhältnis 1:1,9 bei einer Temperatur zwischen 45 °C und 50 °C gemischt. Als Polymerisationsinitiator wurde Locerin (BASF) mit 0,45 % verwendet. Das piezochrome Material wurde mit einer Schichtdicke von 30 µm zwischen jeweils zwei 12 µm dicken Zeonorfolien montiert. Beide Zeonorfolien wurden mit einer Maleinsäureanhydrid-Styren-Copolymer-Orientierungsschicht (< 1 µm) versehen. Das System wurde während 20 min im UV-Licht polymerisiert. Anschließend ließen sich die Zeonorfolien leicht entfernen. Im Ergebnis wurde eine 30 µm dicke piezochrome Schicht erhalten. Bei einer Druckerhöhung von 0,8 bar änderte sie ihre Farbe von rot nach blau in weniger als 1 s.

## Patentansprüche

1. Medizinische Vorrichtung zum visuellen Darstellen eines Injektionsdruckes eines Fluides, wobei die Vorrichtung einen Fluidpfad für das Fluid und ein piezochromes Material aufweist, das bei Einwirkung eines Druckes von < 100 bar eine für das menschliche Auge wahrnehmbare piezochrome Farbänderung zeigt, **dadurch gekennzeichnet, dass** die medizinische Vorrichtung eine erste Konnektoreinrichtung zum Verbinden der medizinischen Vorrichtung mit einem Injektionsschlauch und eine zweite Konnektoreinrichtung zum Verbinden der medizinischen Vorrichtung mit einem Spritzenkörper aufweist.

2. Medizinische Vorrichtung nach Anspruch 1, **dadurch gekennzeichnet, dass** das piezochrome Material bei Einwirkung eines Druckes von < 50 bar, insbesondere < 10 bar, bevorzugt < 5 bar, besonders bevorzugt von 1 bar bis 3 bar, eine für das menschliche Auge wahrnehmbare piezochrome Farbänderung zeigt.

3. Medizinische Vorrichtung nach Anspruch 1 oder 2, **dadurch gekennzeichnet, dass** das piezochrome Material die piezochrome Färbänderung innerhalb eines Zeitraumes von < 1 s zeigt.

4. Medizinische Vorrichtung nach einem der vorhergehenden Ansprüche, **dadurch gekennzeichnet, dass** das piezochrome Material eine Matrix aus wenigstens einem Polymer und/oder Copolymer, wenigstens einen Flüssigkristall und wenigstens eine optisch aktive Substanz aufweist, wobei die optisch aktive Substanz in der Matrix verteilt vorliegt.

5. Medizinische Vorrichtung nach Anspruch 4, **dadurch gekennzeichnet, dass** der wenigstens eine Flüssigkristall ausgewählt ist aus der Gruppe bestehend aus N-(p-Ethoxybenzyliden)-p-n-butylanilin, N-(p-Methoxybenzyliden)-p-n-butylanilin, 4-N-Alkylbenzoesäure-(4-alkylphenylester), Benzoesäurecholesterylester, Cholesterin, Tolan, Alkansäuren, Stilben, Azobenzol, 4-Phenylzimtsäure, p-Terphenyl, 1,2-Bisbenzoethylen, Mischungen aus Alkylcyanobiphenylen und Alkoxycyanobiphenylen und Mischungen von wenigstens zwei der vorgenannten Flüssigkristalle.

6. Medizinische Vorrichtung nach Anspruch 4 oder 5, **dadurch gekennzeichnet, dass** die wenigstens eine optisch aktive Substanz ausgewählt ist aus der Gruppe bestehend aus 4-[[4-(Hexoyl)benzoyl]oxy]-benzoesäure-2-octylester, Cholesterylderivate und Mischungen davon.

7. Medizinische Vorrichtung nach einem der Ansprüche 4 bis 6, **dadurch gekennzeichnet, dass** das Polymer und/oder Copolymer ein Poly(meth)acrylat oder ein Copoly(meth)acrylat ist, das bevorzugt aus wenigstens einem monofunktionalen Monomer und/oder wenigstens einem mehrfach funktionalen Vernetzer aufgebaut ist.

8. Medizinische Vorrichtung nach Anspruch 7 **dadurch gekennzeichnet, dass** das Monomer ausgewählt ist aus der Gruppe bestehend aus Benzyl(meth)acrylat, 2-Ethylhexyl(meth)acrylat, Methoxyethyl(meth)acrylat, Octadecyl(meth)acrylat und Mischungen von wenigstens zwei der vorgenannten Monomere und/oder der Vernetzer ausgewählt ist aus der Gruppe bestehend aus 1,4-Butandioldi(meth)acrylat, Polyethylenglykoldiacrylat, Polyethylenglykoldi(meth)acrylat, 1,10-Decandioldi(meth)acrylat, Bisphenol A-Glycerolat-(1-glycerol)phenoldiacrylat und Mischungen von wenigstens zwei der vorgenannten Vernetzer.

9. Medizinische Vorrichtung nach einem der vorhergehenden Ansprüche, **dadurch gekennzeichnet, dass** das piezochrome Material in einer den Fluidpfad umgebenden Gehäusewandung der medizinischen Vorrichtung enthalten ist.

10. Medizinische Vorrichtung nach Anspruch 9, **dadurch gekennzeichnet, dass** die Gehäusewandung wenigstens einen mehrschichtig aufgebauten Wandungsabschnitt aufweist, wobei eine Zwischenschicht des wenigstens einen mehrschichtig aufgebauten Wandungsabschnittes das piezochchrome Material aufweist oder aus dem piezochromen Material besteht.

11. Medizinische Vorrichtung nach Anspruch 10, **dadurch gekennzeichnet, dass** der wenigstens eine mehrschichtig aufgebaute Wandungsabschnitt ferner eine Innenschicht aufweist, die die Zwischenschicht auf einer dem Fluidpfad zugewandten Seite bedeckt und ein biokompatibles Material aufweist oder aus einem solchen Material besteht.

12. Medizinische Vorrichtung nach Anspruch 11, **dadurch gekennzeichnet, dass** das biokompatible Material ausgewählt ist aus der Gruppe bestehend aus Polyethylenterephthalat, Polyetheretherketon, Polypropylen, Polyphenylsulfon, Polyoxymethylen und Mischungen von wenigstens zwei der vorgenannten biokompatiblen Materialien.

13. Medizinische Vorrichtung nach einem der Ansprüche 10 bis 12, **dadurch gekennzeichnet, dass** der wenigstens eine mehrschichtig aufgebaute Wandungsabschnitt ferner eine Außenschicht aufweist, die die Zwischenschicht auf einer dem Fluidpfad abgewandten Seite bedeckt und ein transparentes Material aufweist oder aus einem transparenten Material besteht.

14. Medizinische Vorrichtung nach Anspruch 13, **dadurch gekennzeichnet, dass** das transparente Material ausgewählt ist aus der Gruppe bestehend aus Polycarbonat, Polymethylmethacrylat, Polyurethan und Mischungen von wenigstens zwei der vorgenannten transparenten Materialien.

## Claims

1. A medical device for visually displaying an injection pressure of a fluid, wherein the device comprises a fluid path for the fluid and a piezochromic material which exhibits a piezochromic color change perceptible to the human eye when exposed to a pressure of < 100 bar, **characterized in that** the medical device comprises a first connector mechanism for connecting the medical device to an injection tube and a second connector mechanism for connecting the medical device to a syringe body.

2. The medical device as claimed in claim 1, **characterized in that** the piezochromic material exhibits a piezochromic color change perceptible to the human eye when exposed to a pressure of < 50 bar, in particular < 10 bar, preferably < 5 bar, particularly preferably from 1 bar to 3 bar.

3. The medical device as claimed in claim 1 or 2, **characterized in that** the piezochromic material exhibits the piezochromic color change within a period of < 1 s.

4. The medical device as claimed in any of the preceding claims, **characterized in that** the piezochromic material comprises a matrix composed of at least one polymer and/or copolymer, at least one liquid crystal and at least one optically active substance, wherein the optically active substance is distributed in the matrix.

5. The medical device as claimed in claim 4, **characterized in that** the at least one liquid crystal is selected from the group consisting of N-(p-ethoxybenzylidene)-p-n-butylaniline, N-(p-methoxybenzylidene)-p-n-butylaniline, 4-alkylphenyl 4-N-alkylbenzoate, cholesteryl benzoate, cholesterol, tolan, alkanoic acids, stilbene, azobenzene, 4-phenylcinnamic acid, p-terphenyl, 1,2-bisbenzoethylene, mixtures of alkylcyanobiphenyls and alkoxycyanobiphenyls, and mixtures of at least two of the aforementioned liquid crystals.

6. The medical device as claimed in claim 4 or 5, **characterized in that** the at least one optically active substance is selected from the group consisting of 2-octyl 4-[[4-(hexoyl)benzoyl]oxy]benzoate, cholesteryl derivatives and mixtures thereof.

7. The medical device as claimed in any of claims 4 to 6, **characterized in that** the polymer and/or copolymer is a poly(meth)acrylate or a copoly(meth)acrylate that has preferably been synthesized from at least one monofunctional monomer and/or at least one multifunctional crosslinker.

8. The medical device as claimed in claim 7, **characterized in that** the monomer is selected from the group consisting of benzyl (meth)acrylate, 2-ethylhexyl (meth)acrylate, methoxyethyl (meth)acrylate, octadecyl (meth)acrylate and mixtures of at least two of the aforementioned monomers and/or the crosslinker is selected from the group consisting of 1,4-butanediol di(meth)acrylate, polyethylene glycol diacrylate, polyethylene glycol di(meth)acrylate, 1,10-decanediol di(meth)acrylate, bisphenol A glycerolate (1 glycerol/phenol) diacrylate and mixtures of at least two of the aforementioned crosslinkers.

9. The medical device as claimed in any of the preceding claims, **characterized in that** the piezochromic material is contained in a housing wall of the medical device that surrounds the fluid path.

10. The medical device as claimed in claim 9, **characterized in that** the housing wall comprises at least one multilayer wall section, wherein an intermediate layer of the at least one multilayer wall section comprises the piezochromic material or consists of the piezochromic material.

11. The medical device as claimed in claim 10, **characterized in that** the at least one multilayer wall section also comprises an inner layer which covers the intermediate layer on a side facing the fluid path and which comprises a biocompatible material or consists of such a material.

12. The medical device as claimed in claim 11, **characterized in that** the biocompatible material is selected from the group consisting of polyethylene terephthalate, polyetheretherketone, polypropylene, polyphenylsulfone, polyoxymethylene and mixtures of at least two of the aforementioned biocompatible materials.

13. The medical device as claimed in any of claims 10 to 12, **characterized in that** the at least one multilayer wall section also comprises an outer layer which covers the intermediate layer on a side facing away from the fluid path and which comprises a transparent material or consists of a transparent material.

14. The medical device as claimed in claim 13, **characterized in that** the transparent material is selected from the group consisting of polycarbonate, polymethyl methacrylate, polyurethane and mixtures of at least two of the aforementioned transparent materials.

## Revendications

1. Dispositif médical pour représenter visuellement une pression d'injection d'un fluide, le dispositif présentant un trajet de fluide pour le fluide et un matériau piézochrome qui présente un changement de couleur piézochrome perceptible par l'œil humain sous l'effet d'une pression < 100 bar, **caractérisé en ce que** le dispositif médical présente un premier appareil connecteur pour relier le dispositif médical à un tube d'injection et un deuxième appareil connecteur pour relier le dispositif médical à un corps de seringue.

2. Dispositif médical selon la revendication 1, **caractérisé en ce que** le matériau piézochrome présente un changement de couleur piézochrome perceptible par l'œil humain sous l'effet d'une pression < 50 bar, notamment < 10 bar, de préférence < 5 bar, de manière particulièrement préférée de 1 bar à 3 bar.

3. Dispositif médical selon la revendication 1 ou 2, **caractérisé en ce que** le matériau piézochrome présente le changement de couleur piézochrome dans une période de temps < 1 seconde.

4. Dispositif médical selon l'une quelconque des revendications précédentes, **caractérisé en ce que** le matériau piézochrome présente une matrice d'au moins un polymère et/ou copolymère, au moins un cristal liquide et au moins une substance optiquement active, la substance optiquement active étant présente de manière dispersée dans la matrice.

5. Dispositif médical selon la revendication 4, **caractérisé en ce que** l'au moins un cristal liquide est choisi dans le groupe constitué par la N-(p-éthoxybenzylidène)-p-n-butylaniline, la N-(p-méthoxybenzylidène)-p-n-butylaniline, les esters (4-alkylphényliques) de l'acide 4-N-alkylbenzoïque, l'ester cholestérique de l'acide benzoïque, le cholestérol, le tolane, les acides alcanoïques, le stilbène, l'azobenzène, l'acide 4-phénylcinnamique, le p-terphényle, le 1,2-bisbenzoéthylène, les mélanges d'alkylcyanobiphényles et d'alkoxycyanobiphényles et les mélanges d'au moins deux des cristaux liquides susmentionnés.

6. Dispositif médical selon la revendication 4 ou 5, **caractérisé en ce que** l'au moins une substance optiquement active est choisie dans le groupe constitué par l'ester 2-octylique de l'acide 4-[[4-(hexoyl)benzoyl]oxy]-benzoïque, les dérivés de cholestéryle et leurs mélanges.

7. Dispositif médical selon l'une quelconque des revendications 4 à 6, **caractérisé en ce que** le polymère et/ou copolymère est un poly(méth)acrylate ou un copoly(méth)acrylate, de préférence formé d'au moins un monomère monofonctionnel et/ou d'au moins un agent de réticulation polyfonctionnel.

8. Dispositif médical selon la revendication 7 **caractérisé en ce que** le monomère est choisi dans le groupe constitué par le (méth)acrylate de benzyle, le (méth)acrylate de 2-éthylhexyle, le (méth)acrylate de méthoxyéthyle, le (méth)acrylate d'octadécyle et les mélanges d'au moins deux des monomères susmentionnés et/ou l'agent de réticulation est choisi dans le groupe constitué par le di(méth)acrylate de 1,4-butanediol, le diacrylate de polyéthylèneglycol, le di(méth)acrylate de polyéthylèneglycol, le di(méth)acrylate de 1,10-décanediol, le diacrylate de bisphénol A-glycérol-(1-glycérol) phénol et les mélanges d'au moins deux des agents de réticulation susmentionnés.

9. Dispositif médical selon l'une quelconque des revendications précédentes, **caractérisé en ce que** le matériau piézochrome est contenu dans une paroi de boîtier du dispositif médical entourant le trajet de fluide.

10. Dispositif médical selon la revendication 9, **caractérisé en ce que** la paroi du boîtier présente au moins une section de paroi à structure multicouche, une couche intermédiaire de l'au moins une section de paroi à structure multicouche présentant le matériau piézochrome ou étant constituée du matériau piézochrome.

11. Dispositif médical selon la revendication 10, **caractérisé en ce que** l'au moins une partie de paroi à structure multicouche présente en outre une couche intérieure qui recouvre la couche intermédiaire sur un côté faisant face au trajet de fluide et présente un matériau biocompatible ou est constituée d'un tel matériau.

12. Dispositif médical selon la revendication 11, **caractérisé en ce que** le matériau biocompatible est choisi dans le groupe constitué par le polyéthylène téréphtalate, la polyétheréthercétone, le polypropylène, la polyphénylsulfone, le polyoxyméthylène et les mélanges d'au moins deux des matériaux biocompatibles susmentionnés.

13. Dispositif médical selon l'une quelconque des revendications 10 à 12, **caractérisé en ce que** l'au moins une partie de paroi à structure multicouche présente en outre une couche extérieure qui recouvre la couche intermédiaire sur un côté détourné du trajet du fluide et présente un matériau transparent ou est constituée d'un matériau transparent.

14. Dispositif médical selon la revendication 13, **caractérisé en ce que** le matériau transparent est choisi dans le groupe constitué par le polycarbonate, le polyméthacrylate de méthyle, le polyuréthane et les mélanges d'au moins deux des matériaux transparents susmentionnés.
